# EUROPÄISCHE PATENTANMELDUNG

(11) **EP 0 675 111 A2**
(43) Veröffentlichungstag der Anmeldung: **04.10.1995**
(21) Anmeldenummer: 95104361.1
(22) Anmeldetag: 24.03.1995
(51) Int. Cl.: C07D 215/14

(54) **Verfahren zur Herstellung von 6-Hydroxymethylchinolinen**

(30) Priorität: 02.04.1994 DE 4411657
(71) Anmelder: BASF AKTIENGESELLSCHAFT, D-67056 Ludwigshafen (DE)
(72) Erfinder: Karrer, Lothar, Dr., D-64319 Pfungstadt (DE); Eichhorn, Hans-Dieter, Dr., D-67273 Weisenheim am Berg (DE); Köhler, Ulrich, Dr., D-68259 Mannheim (DE); Hoffmann, Werner, Dr., D-67141 Neuhofen (DE); Witzel, Tom, Dr., D-67069 Ludwigshafen (DE); Voit, Guido, Dr., D-69198 Schriesheim (DE); Jarret, Robin Stuart, Low Worsall Cleveland Ts15 90 (GB)

(57) **Zusammenfassung**

Verfahren zur Herstellung von 6-Hydroxymethylchinolinen der allgemeinen Formel I 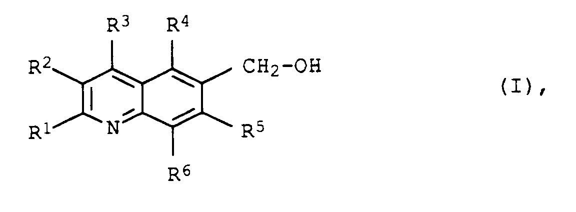
in der
- R¹,R²,R³,R⁴,R⁵,R⁶: Wasserstoff, tert.-Butyl, C₁- bis C₂₀-Alkoxy, Aryl, Aryloxy, Halogen und Hydroxy,
bedeuten, indem man 6-Cyanochinoline der allgemeinen Formel II 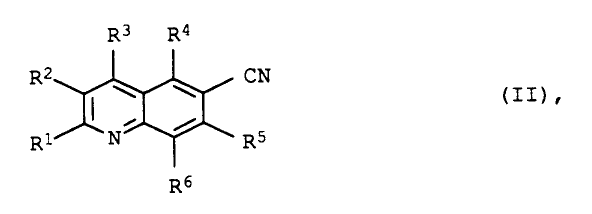
in der R¹, R², R³, R⁴, R⁵ und R⁶ die oben genannten Bedeutungen haben,
a) in Gegenwart eines Hydrierkatalysators bei Temperaturen von 10 bis 100°C und Drücken von 1 bis 200 bar, das entstandene 6-Aminomethylchinolin der allgemeinen Formel III 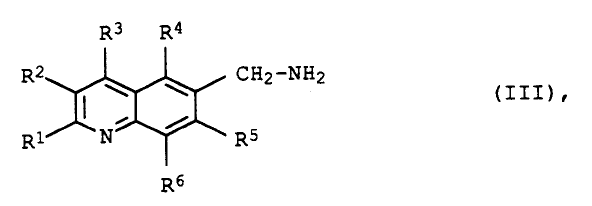 mit einem Diazotierungsmittel in mineralsaurer oder carbonsaurer wäßriger Lösung bei Temperaturen von -10 bis 40°C und Drücken von 0,1 bis 3 bar und gegebenenfalls anschließend das entstandene Acetat mit einer Base bei pH 10 bis 14 und Temperaturen von 10 bis 80°C umsetzt oder
b) in Gegenwart von Wasser und einer Mineralsäure oder einer C₁- bis C₂₀-Carbonsäure und einem Hydrierkatalysator bei Temperaturen von 0 bis 50°C und Drücken von 1 bis 50 bar umsetzt,
sowie die Herstellung der 6-Cyanochinoline durch Umsetzung von 6-Alkylchinolinen der allgemeinen Formel IV 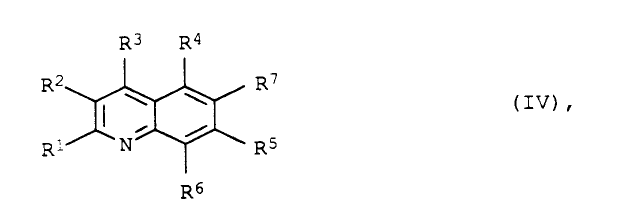
in der R¹, R², R³, R⁴, R⁵ und R⁶ die oben genannten Bedeutungen haben, und R⁷ für C₁- bis C₄-Alkyl steht, mit sauerstoffenthaltenden Gasen und Ammoniak in Gegenwart eines Katalysators, dessen katalytisch aktive Masse 5 bis 95 Gew.-% Vanadium, 5 bis 95 Gew.-% Antimon und 0 bis 20 Gew.-% Alkalimetall enthält, bei Temperaturen von 100 bis 550°C und Drücken von 0,1 bis 50 bar zu den 6-Cyanochinolinen der allgemeinen Formel II.

## Beschreibung

Die vorliegende Erfindung betrifft ein Verfahren zur Herstellung von 6-Hydroxymethylchinolinen durch Umsetzung von 6-Cyanochinolinen
a) in Gegenwart eines Hydrierkatalysators, Diazotierung der entstandenen 6-Aminomethylchinoline in mineralsaurer oder carbonsaurer wäßriger Lösung und gegebenenfalls anschließende Basenbehandlung der entstandenen Acetate oder
b) in Gegenwart von Wasser und einer Mineralsäure oder einer Carbonsäure und einem Hydrierkatalysator,
sowie die Herstellung der verwendeten 6-Cyanochinoline durch Umsetzung von 6-Alkylchinolinen mit sauerstoffenthaltenden Gasen und Ammoniak in Gegenwart eines V/Sb/Alkali-Katalysators.

Aus der EP-A-222 249 ist ein Verfahren zur Herstellung von aromatischen Nitrilen, insbesondere von Phthalsäuredinitrilen, aus entsprechend alkylsubstituierten aromatischen Kohlenwasserstoffen durch katalytische Oxidation mit Sauerstoff und Sauerstoff enthaltenden Gasen in Gegenwart von Ammoniak bei erhöhter Temperatur in der Dampfphase in Gegenwart eines Katalysators, der Vanadiumpentoxid, Antimontrioxid und Alkalioxid sowie Aluminiumoxid enthält, bekannt.

Aus der DE-A-19 48 714 ist ein Verfahren zur Herstellung von Katalysatoren durch Bereitung von Mischungen, die Antimon und Vanadium als Verbindungen oder in elementarer Form und gegebenenfalls eine Trägersubstanz enthalten, und Erhitzen der Mischungen in Gegenwart von Sauerstoff bekannt, deren Mischungen Antimon und Vanadium im molaren Verhältnis von 1,1 : 1 bis 50 : 1 enthalten.

Aus der US-A-3 957 297 ist ein Verfahren zur Herstellung von 3-Cyanpyridin durch Ammonoxidation von 2-Methyl-5-ethylpyridin durch Reaktion mit Ammoniak und Sauerstoff in der Gasphase bei erhöhter Temperatur bekannt, in dem ein Katalysator bestehend aus einem Träger mit niedriger Oberfläche, auf dem Vanadiumoxid und andere Metalloxide abgeschieden worden sind, verwendet wird, wobei die Reaktion in Anwesenheit von wasserdampf durchgeführt wird.

Aus der DE-A-12 90 125 ist ein Verfahren zur Herstellung von Benzonitrilen durch Umsetzung von Alkyl-substituierten Benzolderivaten bekannt in dem die Umsetzung mit Hilfe eines Katalysators aus Antimon- und Vanadiumoxid, gegebenenfalls unter Zusatz von Alkaliverbindungen bei einer Temperatur von 250 bis 500°C durchführt. Es werden z.B. bei der Ammonoxidation von m-Xylol Ausbeuten bis zu 86,1 % Isophthalodinitril an diesem Katalysatorsystem erreicht.

Aus Liebigs Ann. Chem. (1979) 1952 bis 1959 und J. Med. Chem. 13 (1970), 878 bis 882 ist die Reduktion von Cyano-Gruppen in Nachbarstellung zu einem aromatischen System in die entsprechende Alkohole durch hydrolysierende Hydrierung in hochkonzentrierten Mineralsäuren bekannt.

Aus Chem. Abstr. Vol 103, 215195, ist die Herstellung von 2-Hydroxymethylpyrridin aus 2-Cyanopyrridin als 18%ige Lösung in halbkonzentrierter Schwefelsäure an Raney-Nickel bei 20 bar und 30°C mit einer Ausbeute von 69 % bekannt. Das als Nebenprodukt entstehende 2-Aminomethylpyrridin wird in einem weiteren Schritt durch Diazotierung und anschließender Verkochung ebenfalls zum Alkohol umgewandelt, wodurch sich die Ausbeute auf 83,5 % erhöht.

Aus der EP-A-369 208 ist die Herstellung von 2-Hydroxymethyl-3,5-dimethyl-4-methoxypyridin aus der entsprechenden Cyanoverbindung bekannt. Die Cyanoverbindung wird als 3% Lösung in Methanol an Raney-Nickel 3 Tage bei Raumtemperatur und Normaldruck hydriert, das erhaltene Amin durch Destillation isoliert und anschließend in 10%iger Essigsäure bei 0°C mit Natriumnitrit diazotiert und verkocht. Die Gesamtausbeute an destilliertem Alkohol und die Raum-Zeit-Ausbeute ist unbefriedigend.

In DE-A-35 09 618 und EP-A-381 375 wird die Herstellung von 6-Hydroxymethylchinolin aus Chinolin-6-carbonsäureester durch Reduktion mit komplexen Aluminiumhydriden beschrieben. Die Nachteile dieses Verfahrens liegen in dem langen Syntheseweg (Carbonsäure - Carbonsäureester - Alkohol), dem teuren Reduktionsmittel und zusätzlich in der problematischen Aufarbeitung durch die anfallenden Hydrolyseprodukte des Reduktionsmittels.

Der vorliegenden Erfindung lag daher die Aufgabe zugrunde, den zuvor genannten Nachteilen abzuhelfen.

Demgemäß wurde ein neues und verbessertes Verfahren zur Herstellung von 6-Hydroxymethylchinolinen der allgemeinen Formel I 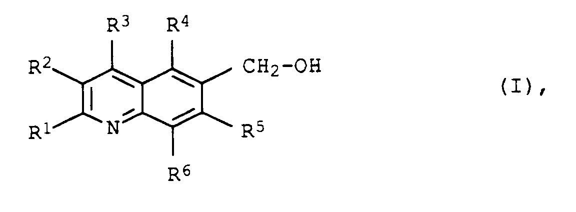
in der
- R¹,R²,R³,R⁴,R⁵,R⁶: Wasserstoff, tert.-Butyl, C₁- bis C₂₀-Alkoxy, Aryl, Aryloxy, Halogen und Hydroxy,
bedeuten, gefunden, welches dadurch gekennzeichnet ist, daß man 6-Cyanochinoline der allgemeinen Formel II 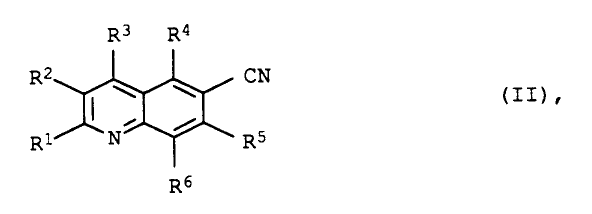
in der R¹, R², R³, R⁴, R⁵ und R⁶ die oben genannten Bedeutungen haben,
a) in Gegenwart eines Hydrierkatalysators bei Temperaturen von 10 bis 100°C und Drücken von 1 bis 200 bar, das entstandene 6-Aminomethylchinolin der allgemeinen Formel III 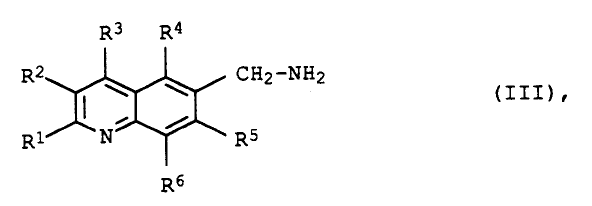 mit einem Diazotierungsmittel in mineralsaurer oder carbonsaurer wäßriger Lösung bei Temperaturen von -10 bis 40°C und Drücken von 0,1 bis 3 bar und gegebenenfalls anschließend das entstandene Acetat mit einer Base bei pH 10 bis 14 und Temperaturen von 10 bis 80°C umsetzt oder
b) in Gegenwart von Wasser und einer Mineralsäure oder einer C₁-bis C₂₀-Carbonsäure und einem Hydrierkatalysator bei Temperaturen von 0 bis 50°C und Drücken von 1 bis 50 bar umsetzt,
sowie die Herstellung der 6-Cyanochinoline durch Umsetzung von 6-Alkylchinolinen der allgemeinen Formel IV 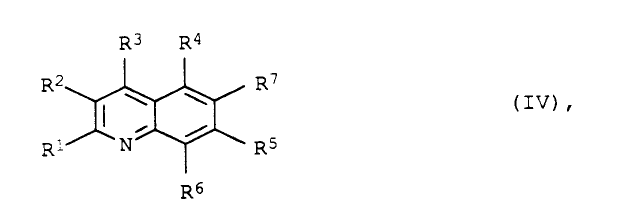
in der R¹, R², R³, R⁴, R⁵ und R⁶ die oben genannten Bedeutungen haben, und R⁷ für C₁- bis C₄-Alkyl steht, mit sauerstoffenthaltenden Gasen und Ammoniak in Gegenwart eines Katalysators, dessen katalytisch aktive Masse 5 bis 95 Gew.-% Vanadium, 5 bis 95 Gew.-% Antimon und 0 bis 20 Gew.-% Cäsium enthält, bei Temperaturen von 100 bis 550°C und Drücken von 0,1 bis 50 bar zu den 6-Cyanochinolinen der allgemeinen Formel II.

Das erfindungsgemäße Verfahren wird wie folgt durchgeführt:

### A) Umsetzung von 6-Cyanochinolinen II

a) Die Cyanochinoline II können in die Hydrierung in Lösung oder Suspension eingesetzt werden.
   Die heterogen, bevorzugt suspensionskatalysierte Hydrierung kann bei Drücken von 1 bis 200 bar, bevorzugt 5 bis 80 bar, besonders bevorzugt 30 bis 60 bar und Temperaturen von 10 und 100°C, bevorzugt 30 bis 70°C durchgeführt werden.
   Die Hydrierung kann bevorzugt unter Zusatz von Ammoniak zum 6-Cyanochinolin von 0,5 : 1 bis 10 : 1, bevorzugt 1 : 1 bis 2 : 1 durchgeführt werden.
   Nach Abtrennen des Katalysators und Abdestillieren des Ammoniaks und des Lösungsmittels kann das erhaltene Aminomethylchinolin aufdestilliert und somit in reiner Form gewonnen werden. Bevorzugt wird das Produkt ohne weitere Aufarbeitung in die Diazotierung eingesetzt.
   Die Diazotierung/Verkochung kann bei Temperaturen von -10 bis 40°C, bevorzugt 5 bi 20°C, besonders bevorzugt 5 bis 15°C und Drücken von 0,01 bis 5 bar, bevorzugt 0,1 bis 2 bar, besonders bevorzugt Normaldruck (Atmosphärendruck) mit einem Diazotierungsmittel durchgeführt werden.
   Als Diazotierungsmittel eignen sich Natriumnitrit, Silbernitrit, Silbernitrit oder Alkylnitrite, bevorzugt wäßriges Natriumnitrit in mineralsaurer oder carbonsaurer wäßriger Lösung, besonders bevorzugt in Essigsäure.
   Die Konzentration der Mineralsäure oder Carbonsäure in wäßriger Lösung beträgt in der Regel 10 bis 80 Gew.-%, bevorzugt 10 bis 30 Gew.-%, besonders bevorzugt 15 bis 25 Gew.-%.
   Anschließend kann die Reaktionsmischung durch Einstellen mit einer organischen oder anorganischen Base wie Tetraalkylammoniumhydroxid, Kalilauge und Natronlauge, bevorzugt Kalilauge und Natronlauge, besonders bevorzugt Natronlauge auf einen pH-Wert zwischen 10 bis 14 bei einer Temperatur zwischen 30 bis 80°C, bevorzugt 40 bis 60°C verseift werden.
b) Die 6-Cyanochinoline II können in Lösung oder Suspension eingesetzt werden. Als Lösungs- oder Suspensionsmittel kommen wäßrige Mineralsäuren wie Salzsäure, Schwefelsäure und Phosphorsäure oder wäßrige organische Säuren wie p-Toluolsulfonsäure, Ameisensäure, Essigsäure oder Gemische aus diesen, bevorzugt wäßrige organische Säuren, besonders bevorzugt wäßrige Essigsäure zum Einsatz. Die Säurekonzentration liegt in der Regel bei 10 bis 90 Gew.-%, bevorzugt 20 bis 50 Gew.-%. Die Säure kann auch in Form eines sauren Ionenaustauschers zugegeben werden.
   Die Hydrierung kann in der Regel bei Drücken zwischen 1 und 50 bar, bevorzugt zwischen 1 und 20 bar, besonders bevorzugt bei Normaldruck (Atmosphärendruck) und bei Temperaturen zwischen 0 und 50°C, bevorzugt zwischen 10 und 20°C durchgeführt werden.

Als Hydrierkatalysatoren (Reduktionskatalysatoren) kommen für a) und b) alle gängigen Hydrierkatalysatoren in Frage. Die Reaktion kann entweder suspensionskatalysiert z.B. an Pd/C, Rh/C, Ru/C, Re/C, Pt/C, Raney-Nickel oder Raney-Cobalt, bevorzugt Raney-Cobalt, Raney-Nickel oder Pd/C, besonders bevorzugt Pd/C oder Raney-Nickel oder festbettkatalysiert an Edelmetall-Trägerkatalysatoren wie z.B. Pd/C, Pd/Al₂0₃, Re/TiO₂ oder Ru/C oder Cobalt, Nickel oder Kupfer-Vollkontakten durchgeführt werden. Bevorzugt wird die Suspensionsfahrweise, wobei sich der Suspensionskatalysator mehrfach verwenden läßt.

Als Lösungs- oder Suspensionsmittel in der Hydrierung für a) und b) kommen alle Lösungsmittel in Frage, die unter den Hydrierbedingungen inert sind und den Katalysator nicht vergiften wie z.B. C₅- bis C₃₀-Kohlenwasserstoffe, C₁- bis C₈-Alkanole wie Methanol und Ethanol, C₁- bis C₂₀-Alkylformamide wie Dimethylformamid, C₂- bis C₂₀-Ether, bevorzugt cyclische Ether wie Tetrahydrofuran oder acyclische Ether wie Methyl-tert.-butylether und Methyl-iso-propylether, Carbonsäuren wie Ameisensäure, Essigsäure und Propionsäure, Carbonsäureester wie Essigsäuremethylester und Essigsäureethylester, cyclische Amide wie N-Methylpyrrolidon.

Die Aufarbeitung des Rohalkohols nach a) oder b) kann entweder durch Extraktion oder durch Destillation erfolgen.

Bei der Extraktion können zuerst bei pH 2 ungewünschte Nebenprodukte, und anschließend bei pH 7 der gewünschte Alkohol in hoher Reinheit (> 95%) extrahiert werden. Das Nebenprodukt 6-Aminomethylchinolin wird bei diesem pH-Wert nicht mitextrahiert, kann jedoch durch anschließende Extraktion bei pH 14 ebenfalls in hoher Reinheit (> 95%) erhalten werden.

Als Extraktionsmittel kommen Ether, Ketone oder aromatische oder aliphatische Kohlenwasserstoffe in Frage. Bevorzugt werden Methyl-tert.-butylether, Methyl-iso-propylether, Toluol, Cyclohexan, 2-Ethylhexanol, besonders bevorzugt Methyl-tert.-butylether und 2-Ethylhexanol.

Nach Entfernung des Lösungsmittels können die Produkte in kristalliner Form erhalten und anschließend z.B. aus Toluol, Essigester, Methyl-tert.-butylether, Methyl-iso-propylether oder Tetrahydrofuran, besonders bevorzugt Toluol umkristallisiert werden.

### B) Herstellung von 6-Cyanochinolinen II aus 6-Alkylchinolinen IV

Man kann die 6-Alkylchinoline IV mit sauerstoffenthaltenden Gasen und Ammoniak in Gegenwart eines Katalysators, dessen katalytisch aktive Masse Vanadium, Antimon und Cäsium enthält, bei Temperaturen von 100 bis 55°C, bevorzugt 300 bis 520°C, besonders bevorzugt 400 bis 500°C und Drücken von 0,1 bis 50 bar, bevorzugt 0,5 bis 5 bar, besonders bevorzugt Normaldruck (Atmosphärendruck) diskontinuierlich oder kontinuierlich umsetzen. Dazu wird das Gas, welches 6-Alkylchinolin, Sauerstoff, Ammoniak und möglicherweise weitere Komponenten enthält, über einen Katalysator geleitet. Der Katalysator kann in Form von Kugeln, Ringen, weiteren Formkörpern oder Pulver vorliegen, wobei im letzten Fall bevorzugt eine Wirbelschicht gewählt wird.

Als sauerstoffhaltige Gase eignen sich Sauerstoff, Luft, oder ein Gemisch aus Sauerstoff und Luft, welches mindestens 2 Mol-% Sauerstoff, bevorzugt mindestens 5 Mol-% und besonders bevorzugt mindestens 8 Mol-% Sauerstoff enthält.

Als Katalysatoren deren katalytisch aktive Masse Vanadium, Antimon und Alkalimetall enthält, eignen sich Katalysatoren mit einer katalytisch aktive Masse mit 5 bis 95 Gew.-% Vanadium, bevorzugt 10 bis 90 Gew.-%, besonders bevorzugt 10 bis 80 Gew.-%, 5 bis 95 Gew.-%, bevorzugt 10 bis 90 Gew.-%, besonders bevorzugt 10 bis 80 Gew.-% Antimon und 0 bis 20 Gew.-% eines Alkalimetalls, bevorzugt 0 bis 10 Gew.-%, besonders bevorzugt 0 bis 5 Gew.-% enthält.

Unter den Alkalimetallen eignen sich Lithium, Natrium, Kalium, Ruthenium und Cäsium, bevorzugt Natrium, Kalium und Cäsium, besonders bevorzugt Cäsium.

Man kann diese Katalysatoren als Vollkontakte oder bevorzugt auf einem Träger, bevorzugt einem porösen Träger aufgebracht verwenden. Die katalytisch aktive Masse wird in der Regel in Form von Oxiden auf den Träger z.B. durch Tränken, Imprägnieren oder Aufsprühen aufgebracht.

Als Träger eignen sich Titandioxid, Aluminiumoxid, Siliciumdioxid oder Zirkondioxid oder ein Gemisch davon.

Die Trägerkatalysatoren haben in der Regel eine BET-Oberfläche von 10 bis 350 m²/g, bevorzugt 40 bis 250 m²/g, besonders bevorzugt 60 bis 200 m²/g. Falls die Wirbelschichtvariante gewählt wird, beträgt die mittlere Partikelgröße in der Regel 20 bis 500 µm, bevorzugt 50 bis 350 µm, besonders bevorzugt 70 bis 180 µm, wobei in der Regel weniger als 10 Gew-% der Teilchen einen Durchmesser unter 30 µm und in der Regel weniger als 10 Gew.-% der Teilchen einen Durchmesser über 200 µm haben. Der Hauptteil der Poren liegt in der Regel im Bereich zwischen 2 nm und 1 µm.

Bei imprägnierten Trägerkatalysatoren kann der Aktivmassenvorläufer bestehend aus Lösungen von Komplexsalzen wie Antimon-Tartrat und Vanadium-Oxalat sowie aus einer Alkalisalzlösung z.B. durch Eintauchen und inniges Vermischen auf den Träger aufgebracht werden. Das feuchte Pulver wird in der Regel mehrere Stunden 2 bis 10 h bei erhöhten Temperaturen von 60 bis 200°C, bevorzugt 80 bis 180°C, besonders bevorzugt 100 bis 140°C getrocknet und anschließend mehrere Stunden 2 bis 10 h bei erhöhten Temperaturen von 300 bis 900°C, bevorzugt 400 bis 750°C, besonders bevorzugt 450 bis 700°C 3 h in einem Ofen, Drehrohr oder Wirbelbett calciniert. Das so erhaltene calcinierte Katalysatorpulver kann in einem Wirbelschichtreaktor für die Synthese verwendet werden.

Die Substituenten R¹, R², R³, R⁴, R⁵, R⁶ und R⁷ in den Verbindungen I, II, III und IV haben folgende Bedeutungen:
- R¹,R²,R³,R⁴,R⁵,R⁶,R⁷: - unabhängig voneinander
- Wasserstoff,
- tert.-Butyl,
- C₁- bis C₂₀-Alkoxy, bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁- bis C₄-Alkoxy wie Methoxy, Ethoxy, n-Propoxy, iso-Propoxy, n-Butoxy, iso-Butoxy, sec.-Butoxy und tert.-Butoxy,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- Aryloxy wie Phenoxy, 1-Naphthoxy, 2-Naphthoxy, 1-Anthroxy, 2-Anthroxy und 9-Anthroxy, bevorzugt Phenoxy, 1-Naphthoxy und 2-Naphthoxy, besonders bevorzugt Phenoxy,
- Halogen wie Fluor, Chlor, Brom und Jod, bevorzugt Fluor, Chlor und Brom, besonders bevorzugt Fluor und Chlor und
- Hydroxy,
- R⁷: - C₁- bis C₄-Alkyl wie Methyl, Ethyl, n-Propyl, iso-Propyl, n-Butyl, iso-Butyl, sec.-Butyl und tert.-Butyl, bevorzugt C₁- bis C₃-Alkyl wie Methyl, Ethyl, n-Propyl und iso-Propyl, besonders bevorzugt Methyl und Ethyl,
besonders bevorzugt sind die Verbindungen I, II, III und IV in denen R¹, R², R³, R⁴, R⁵ und R⁶ gleichzeitig Wasserstoff bedeuten. 6-Hydroxymethylchinoline sind Zwischenprodukte für die Synthese von Pharma-Wirkstoffen (DE-A-35 09 618, EP-A-381 375).

### Beispiele

### A) Umsetzung von 6-Cyanochinolinen II

### Variante a)

### Beispiel 1

100 g 6-Cyanochinolin wurden in 500 ml Tetrahydrofuran suspendiert und in Gegenwart von 500 ml Ammoniak und 20 g Raney-Nickel bei 50 bar und 50°C 10 h hydriert. Nach 3 h war die Wasserstoffaufnahme beendet. Ammoniak und Tetrahydrofuran wurden abdestilliert, und das erhaltene 6-Aminomethylchinolin in 2200 g Essigsäure (50%ig) gelöst und 48 g Natriumnitrit, gelöst in 250 ml Wasser, innerhalb 40 Minuten bei 10°C zugetropft. Anschließend wurde mit 20%iger Natronlauge auf pH 14 gestellt und 2 h bei 60°C gerührt. Durch Extraktion mit Methyl-tert.-butylether und Destillation erhielt man 71 g (69 %) 6-Hydroxymethyl-chinolin (Sdp.: 195°C/10 mbar; Smp.: 74 bis 76°C [79 bis 80°C nach J. Org. Chem. **18** (1953) 55]).

### Beispiel 2

200 g 6-Cyanochinolin wurden in 500 ml Methanol suspendiert und in Gegenwart von 500 ml Ammoniak und 40 g Raney-Nickel bei 50 bar und 50°C 3 h hydriert. Nach Entfernung von Ammoniak und Methanol wurde das erhaltene 6-Aminomethylchinolin in 800 g Essigsäure (25%ig) gelöst und 90 g Natriumnitrit, gelöst in 200 ml Wasser, innerhalb 60 Minuten bei 10°C zugetropft. Anschließend wurde mit 50%iger Natronlauge auf pH 12 gestellt und 2 h bei 50°C gerührt. Durch Extraktion mit Methyl-tert.-butylether, Entfernung des Lösungsmittels i. Vak. und Umkristallisation aus Toluol erhielt man 148 g (72 %) 6-Hydroxymethylchinolin (Smp.: 75 bis 77°C).

### Variante b)

### Beispiel 3

40 g 6-Cyanochinolin wurden in 500 ml Wasser und 500 ml Essigsäure gelöst und an 20 g Katalysator (10% Palladium auf Kohle) 8 h bei 10°C unter starkem Rühren bei Normaldruck mit Wasserstoff hydriert. Nach Abfiltrieren des Katalysators und Abdestillieren der wäßrigen Essigsäure wurde der ölige Rückstand mit Wasser versetzt, mit konz. Schwefelsäure auf pH 2 gestellt, mit Methyl-tert.-butyl-ether extrahiert und der Extrakt verworfen. Der Extraktionsrück-stand wurde mit 50%iger Natronlauge auf pH 7 gestellt, das 6-Hydroxymethyl-chinolin mit Methyl-tert.-butylether extrahiert und i. Vak. vom Lösungsmittel befreit. Das erhaltene kristalline Rohprodukt wurde mit n-Hexan digeriert. Man erhielt 28,7 g (70 %) 6-Hydroxymethyl-chinolin (Fp.: 76°C).

### Beispiel 4

100 g 6-Cyanochinolin wurden in 250 ml Wasser und 250 ml Essigsäure suspendiert und an 50 g Katalysator (10% Palladium auf Kohle) 2 h bei 25°C unter starkem Rühren bei Normaldruck mit Wasserstoff hydriert. Nach Abfiltrieren des Katalysators und Abdestillieren der wäßrigen Essigsäure wurde der ölige Rückstand mit Wasser versetzt, mit konz. Schwefelsäure auf pH 2 gestellt, mit Methyl-tert.-butylether extrahiert und der Extrakt verworfen. Der Extraktionsrückstand wurde mit 50%iger Natronlauge auf pH 7 gestellt, 6-Hydroxymethylchinolin mit Methyl-t-Butylether extrahiert und i. Vak. vom Lösungsmittel befreit. Das erhaltene kristalline Rohprodukt wurde aus Toluol umkristallisiert. Man erhielt 53,9 g (52 %) 6-Hydroxymethylchinolin (Fp.: 74°C).

### Beispiel 5

75 g 6-Cyanochinolin wurden in 450 ml Wasser und 450 ml Essigsäure suspendiert und an 37 g 5% Palladium auf Kohle 4 h bei 12°C unter starkem Rühren und Normaldruck mit Wasserstoff hydriert. Nach Abfiltrieren des Katalysators und Abdestillieren der wäßrigen Essigsäure und leichtsiedender Nebenkomponenten wurde das 6-Hydroxymethylchinolin bei 5 mbar und einer Sumpftemperatur von 200°C abdestilliert, das so erhaltene kristalline Rohprodukt wurde aus Toluol umkristallisiert. Man erhielt 48,9 g (63 %) 6-Hydroxymethylchinolin (Fp.: 74°C).

### Beispiel 6

Wie Beispiel 3, nur wurde der Katalysator aus Beispiel 3 eingesetzt. Man erhielt:
a) 1. Rückführung: 60 % Ausbeute (isoliert)
b) 2. Rückführung: 58 % Roh-Ausbeute (GC-Gew%)
c) 3. Rückführung: 62 % Roh-Ausbeute (GC-Gew%)

### Beispiel 7

8 g 6-Cyanochinolin wurden in 100 g Wasser/100g Essigsäure gelöst und über eine Rieselsäule - gefüllt mit 40 g Katalysator (4 mm Stränge, 5% Pd auf Kohle) - 5 h im Kreislauf gefahren (70 ml/h, 25°C, Normaldruck). Im Gegenstrom wurden 20 l Wasserstoff/h zugeleitet. Die Ausbeute laut GC-Gew.-% betrug 52%.

### B) Herstellung von 6-Cyanochinolinen II aus 6-Alkylchinolinen IV

### Beispiel 8

105 g/h 6-Methylchinolin wurden verdampft und bei 450°C zusammen mit 780 Nl/h Ammoniak und 60 Nl/h Sauerstoff über den Wirbelschichtreaktor gefahren. Es wurden 650 g Katalysatorpulver eingesetzt. Der Katalysator wurde durch Imprägnieren eines Aluminiumoxidpulvers mit Vanadium-, Antimon- und Cäsiumsalzlösungen hergestellt. Das Rohkatalysatorpulver wurde bei 500°C über 2 Stunden calciniert. Bei der Umsetzung von 6-Methylchinolin wurde eine molare Ausbeute von 80 % 6-Cyanchinolin erhalten. Der Rest waren Totaloxidationsprodukte CO und CO₂ sowie nicht umgesetztes 6-Methylchinolin.

## Patentansprüche

1. Verfahren zur Herstellung von 6-Hydroxymethylchinolinen der allgemeinen Formel I 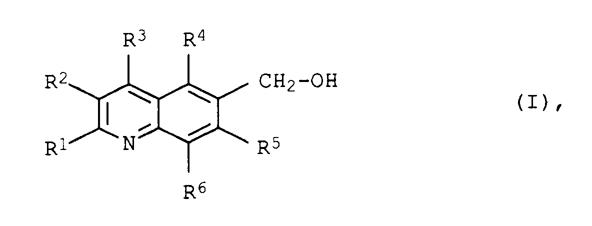 in der
R¹,R²,R³,R⁴,R⁵,R⁶ Wasserstoff, tert.-Butyl, C₁- bis ₂₀-Alkoxy, Aryl, Aryloxy, Halogen und Hydroxy,
bedeuten, dadurch gekennzeichnet, daß man 6-Cyanochinoline der allgemeinen Formel II 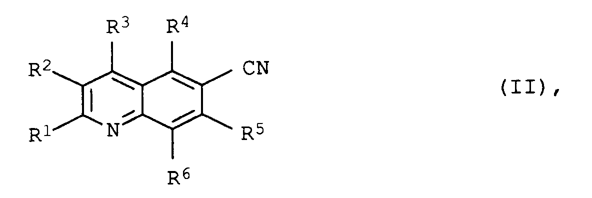 in der R¹, R², R³, R⁴, R⁵ und R⁶ die oben genannten Bedeutungen haben,
a) in Gegenwart eines Hydrierkatalysators bei Temperaturen von 10 bis 100°C und Drücken von 1 bis 200 bar, das entstandene 6-Aminomethylchinolin der allgemeinen Formel III 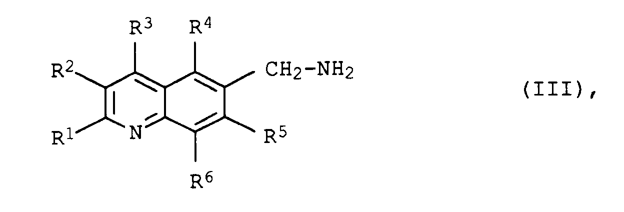 mit einem Diazotierungsmittel in mineralsaurer oder carbonsaurer wäßriger Lösung bei Temperaturen von -10 bis 40°C und Drücken von 0,1 bis 3 bar und gegebenenfalls anschließend das entstandene Acetat mit einer Base bei pH 10 bis 14 und Temperaturen von 10 bis 80°C umsetzt oder
b) in Gegenwart von Wasser und einer Mineralsäure oder einer C₁- bis C₂₀-Carbonsaure und einem Hydrierkatalysator bei Temperaturen von 0 bis 50°C und Drücken von 1 bis 50 bar umsetzt.

2. Verfahren zur Herstellung von 6-Hydroxymethylchinolinen nach Anspruch 1, dadurch gekennzeichnet, daß R¹, R², R³, R⁴, R⁵ und R⁶ gleichzeitig Wasserstoff bedeuten.

3. Verfahren zur Herstellung von 6-Hydroxymethylchinolinen der allgemeinen Formel I 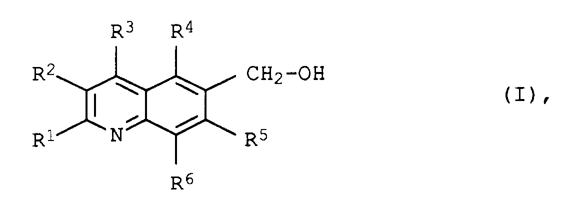 in der
R¹,R²,R³,R⁴,R⁵,R⁶ Wasserstoff, tert.-Butyl, C₁- bis C₂₀-Alkoxy, Aryl, Aryloxy, Halogen und Hydroxy,
bedeuten, dadurch gekennzeichnet, daß man 6-Alkylchinoline der allgemeinen Formel IV 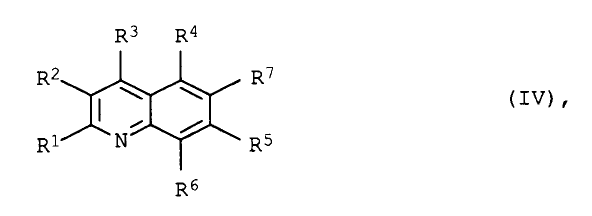 in der R¹, R², R³, R⁴, R⁵ und R⁶ die oben genannten Bedeutungen haben, und R⁷ für C₁- bis C₄-Alkyl steht, mit sauerstoffenthaltenden Gasen und Ammoniak in Gegenwart eines Katalysators, dessen katalytisch aktive Masse 5 bis 95 Gew.-% Vanadium, 5 bis 95 Gew.-% Antimon und 0 bis 20 Gew.-% Alkalimetall enthält, bei Temperaturen von 100 bis 550°C und Drücken von 0,1 bis 50 bar zu den 6-Cyanochinolinen der allgemeinen Formel II 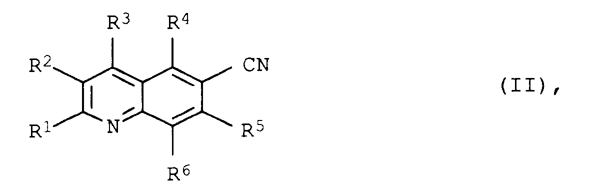 in der R¹, R², R³, R⁴, R⁵ und R⁶ die oben genannten Bedeutungen haben, umsetzt, und diese
a) in Gegenwart eines Hydrierkatalysators bei Temperaturen von 10 bis 100°C und Drücken von 1 bis 200 bar, das entstandene 6-Aminomethylchinolin der allgemeinen Formel III 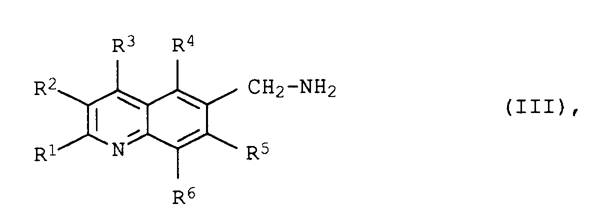 mit einem Diazotierungsmittel in mineralsaurer oder carbonsaurer wäßriger Lösung bei Temperaturen von -10 bis 40°C und Drücken von 0,1 bis 3 bar und gegebenenfalls anschließend das entstandene Acetat mit einer Base bei pH 10 bis 14 und Temperaturen von 10 bis 80°C umsetzt oder
b) in Gegenwart von Wasser und einer Mineralsäure oder einer C₁- bis C₂₀-Carbonsäure und einem Hydrierkatalysator bei Temperaturen von 0 bis 50°C und Drücken von 1 bis 50 bar umsetzt.
